Europäisches Patentamt

**European Patent Office**

**Office européen des brevets**

(11) Publication number: **0 176 314 B1**

(12) EUROPEAN PATENT SPECIFICATION

(45) Date of publication of patent specification: **10.06.92**  (51) Int. Cl.⁵: **A61B 6/00,** G01T 1/166

(21) Application number: **85306633.0**

(22) Date of filing: **18.09.85**

(54) **Radiography system.**

(30) Priority: **21.09.84 US 653955**

(43) Date of publication of application:
**02.04.86 Bulletin 86/14**

(45) Publication of the grant of the patent:
**10.06.92 Bulletin 92/24**

(84) Designated Contracting States:
**DE FR GB NL**

(56) References cited:
**FR-A- 2 549 711**
**US-A- 3 717 762**
**US-A- 4 176 280**
**US-A- 4 383 327**

(73) Proprietor: **PICKER INTERNATIONAL, INC.**
**595 Miner Road**
**Highland Heights Ohio 44143(US)**

(72) Inventor: **Sones, Richard A.**
**3790 Bainbridge Road**
**Cleveland Heights Ohio 44118(US)**
Inventor: **Tesic, Mike M.**
**17838 Landseer**
**Cleveland Ohio 44119(US)**

(74) Representative: **Pope, Michael Bertram Win-**
**gate et al**
**Central Patent Department Wembley Office**
**The General Electric Company, p.l.c. Hirst**
**Research Centre East Lane**
**Wembley Middlesex HA9 7PP(GB)**

EP 0 176 314 B1

**Description**

This invention relates to radiography systems.

Radiography is a long known medical diagnostic imaging technique.

In a conventional radiography system a radiation source, e.g. an x-ray source, is actuated to direct a divergent area beam of x-rays through a patient. A cassette containing an x-ray sensitive phosphor screen and light and x-ray sensitive film is positioned in the x-ray path on the side of the patient opposite the source. X-radiation passing through the patient's body is attenuated to produce a shadow image of a portion of the patient through which the x-rays pass.

More recently, digital radiographic techniques and systems have been developed. In digital radiography, the source directs x-radiation through a patient's body to a detector assembly located in the beam path beyond the patient. The detector produces electrical signals defining the radiation pattern emergent from the patient. These signals are then processed to yield a visual display of the image.

The detector assembly includes an elongated array of individual discrete detector elements. Each detector element responds to incident x-radiation to produce an analogue electrical charge signal indicative of such radiation. These analog electrical signals represent the radiation pattern or image emergent from the patient's body and incident on the detector array.

The analog signals are sampled and processed by imaging circuitry, primarily to improve their signal to noise ratio, and are subsequently digitized.

The digital signals are fed to a digital data processing unit (DPU). The data processing unit records and/or processes and enhances the digital data.

A display unit responds to appropriate digital data representing the image to convert the digital information back into analog form and to produce a visual display of the patient's internal body structure derived from the acquired image pattern of radiation.

The display unit can be coupled directly to the digital data processing unit for substantially real time imaging, or can be fed stored digital data from digital storage means such as tapes or disks representing patient images produced from earlier studies.

Digital radiography includes techniques in which a thin spread beam of x-radiation is used. In practice of this technique, often called "scan (or slit) projection radiography" (SPR), the spread beam is scanned across the patient, or the patient is movably interposed between the spread beam X-ray source and the detector assembly, the detector being maintained in continuous alignment with the beam. The relative movement effected between the source-detector arrangement and the patient's body scans a large portion of the body.

Discrete element detectors have been proposed comprising a single line of detector elements. Other proposals have included rectangular detector arrays of square detector elements.

Details of certain aspects of digital radiography systems such as described here are set forth in the following publications: Mattson, R.A., et al, "Design and Physical Characteristics of a Digital Chest Unit", S.P.I.E. Volume 314, Digital Radiography (1981).

Arnold, B.A. et al "Digital Radiography: An Overview" Proc. of S.P.I.E. Volume 273, March 1981; Kruger, R.A. et al "A Digital Video Image Processor for Real Time X-Ray Subtraction Imaging" Optical Engineering Volume 17, No. 6 (1978);

European Patent Application No. EP-A-0115125, entitled "Split Energy Level Radiation Detection".

An alternate proposal to the detector element array described above is a detector array consisting of two side by side vertical columns of square detector elements. One of the columns, however, is slightly vertically displaced, or offset, with respect to the other by a distance equal to one half the height of a single detector element. Such a configuration is described in the above European application.

It has also been proposed, where the detector array comprises a rectangular array of square detector elements, to improve the signal to noise ratio of the information developed by the detector, by the use of time delay and integrate (TDI) circuitry, as described, for example, in US-A-4,383,327. Such proposed TDI systems employ sampling at regular intervals of detector motion, and motion-synchronous shifting and adding of individual detector-produced analogue charge signals. In such systems, the TDI circuitry can be integral with the detector elements.

Important advantages of scanning slit radiography are excellent scatter rejection and compatibility with digital image sensors. A significant disadvantage of such systems is the requirement of heavy X-ray tube loading that results from inefficient utilization of the X-ray output. This inefficiency arises from the aperture width which defines the spread beam subtending only a small solid angle at the focal spot of the X-ray tube.

To alleviate this disadvantage, it has been proposed to use a spread beam which is as thick as possible

2

without unduly compromising the inherent good scatter rejection of such systems employing thin beams. As the spread beam is widened, however, the use of a rectangular detector array becomes more difficult. Such difficulty arises because, as the spread beam is thickened, more detector elements are needed, data rates become high and accordingly more difficult to handle, and TDI techniques must be used for shifting and adding data synchronously with scan motion so that data pertaining to each image portion, or "pixel", are properly superimposed to avoid image blurring.

Where time delay and integrate shifting and adding circuitry is employed, the detector element output signals are sampled at successive increments of detector movement equal to the length of a side of a single detector element. For reasons explained in more detail below, the spatial resolution of a rectangular detector array when used with TDI as described above is poorer than the maximum inherently obtainable resolution.

To facilitate understanding of both the prior art and the present invention, certain information and definitions relating to imaging optics are useful.

The ability of any optical element or system to resolve images is often described in terms of its "modulation transfer function" (MTF). Normally, the ability of an optical system to resolve a portion of an image decreases as the fineness of detail of the image portion (the number of lines per unit distance) increases. The number of lines per unit distance is frequently expressed as "line pairs per millimeter", and is known as the "spatial frequency" of the image portion of interest. The degradation of resolution as detail increases is manifested as a reduction in the contrast between the light and dark areas of the image portion. MTF is the function of contrast ratio versus the spatial frequency.

A rectangular detector element has an MTF in each of the x and y co-ordinates of its energy receiving face. In a square detector element $MTF_x = MTF_y$, and both functions can be demonstrated to be represented by the expression sinc (pf), where p is the length of one side of the square element receiving face, f is the spatial frequency sought to be imaged, and the sinc function is defined as sinc $x = \sin(\pi x)/(\pi x)$

According to the above relationship, the x and y MTF's each are first reduced to 0 when the spatial frequency increases to $f = 1/p$. This first zero is generally taken as representing the maximum spatial frequency (detail) which a square detector element can reliably image.

This phenonemon is one limiting factor on the resolving capability of any square detector, and is dependent upon its size, or "aperture". This parameter is referred to as the "aperture cutoff frequency".

A detector element is also limited by another resolution constraint known as the "Nyquist frequency". The Nyquist frequency is a spatial frequency above which the detector element cannot resolve separate lines. Rather than being a function of the detector size, however, the Nyquist frequency is related to the incremental distance at which successive samplings of the detector element output signal occur. The Nyquist cutoff frequency is relevant since the use of TDI circuitry requires repeated detector output samplings.

Where a row of square detectors, extending in the x coordinate in a rectangular array, is sampled once for each successive element width increment, (sampling distance) (as in the prior art) it can be shown, as set forth in publications referenced below, that the Nyquist frequency is only $1/(2p)$, along both coordinate axes. Therefore, in such a rectangular array, sampled as described, the Nyquist frequency is twice as limiting to resolution as is the aperture cutoff. Thus, the spatial frequency at which the resolving capability disappears under the Nyquist criterion is only half the frequency at which the resolving capability disappears under the aperture cutoff frequency criterion.

This means that, where a moving rectangular array of square elements is employed, and a row of elements is sampled only at successive increments of one detector width, the spatial resolution of such a detector is poorer than the maximum obtainable, as dictated by the detector element size, or aperture, under the aperture cutoff criterion. Also, aliasing artifacts will be present in an image derived from such a detector.

The Nyquist criterion is also applicable in the y coordinate of a rectangular array. In the y direction, the equivalent sampling distance between adjacent rows of square elements is the length p of one side of an element.

The following publications are explanatory of the theory relating to these conclusions:

Sones, R.A., et al "A Method to Measure the MTF of Digital X-ray Systems" Medical Physics 11(2), March/April 1984, pages 166-171; Giger,M.L. et al "Investigation of Basic Imaging Properties in Digital Radiography: Modulation Transfer Function" Medical Physics 11(3) May/June 1984, at pages 287-295.

It has been seen that, where a rectangular array of square elements is sampled only once for each successive element width of motion, the system fails to take full advantage of the resolving power of the elements as dictated by their size.

The same conclusion applies to resolution in the y coordinate of the rectangular array. This is because the effective sampling distance between adjacent rows of the arrays is defined as p, the same as in the x direction.

Therefore, a rectangular array, sampled at incremental distance p fails to take full advantage of its inherent resolving power, in either the x or y coordinate.

It has been proposed that the use of an offset array, having only two columns, might be used to improve (reduce) the effective sampling distance increment in the y direction. This, however, is only a partial improvement, since it does not afford any reduction in the sampling distance in the x coordinate.

Moreover, since such detectors have only two columns of elements, and the detector array scans perpendicular to the columns, such an array has not been used in conjunction with TDI imaging circuitry. Thus, the signal-enhancing benefits of such circuitry have not been useful in conjunction with any known staggered, or offset, array.

It is an object of this invention to take maximum advantage of the resolving capabilities of the individual detector elements of the detector array, as defined by the aperture cutoff criterion, by eliminating Nyquist frequency restriction in both x and y coordinates, while maintaining the full benefits of the use of time delay and integrate circuitry for enhancing the signal to noise ratio of the data acquired by the detector array.

According to the present invention, a radiography system comprises: a radiation source, a detector array comprising a plurality of rows of uniform detector elements interspersed so as to define a plurality of columns perpendicular to said rows and arranged in a staggered pattern, each detector element being responsive to radiation incident thereon for producing an electrical output signal indicative of said incident radiation; means for mounting said source and said detector array spaced sufficiently from one another to accommodate a body in a space therebetween; mechanism for scanning said detector array relative to said body when located in said space, in a direction substantially parallel to said rows; means for actuating said radiation source to propagate radiation from the source and toward said detector array, said radiation passing through a portion of said body located in said space, and time delay and integrate circuitry coupled to said detector elements and separate therefrom for producing an image of internal body structure in response to said electrical signals produced by said detector elements, said circuitry comprising sampling circuitry for sampling said detector element electrical signals at successive increments of detector array scanning motion characterised in that said successive increments are less than a distance equal to a dimension of one of said uniform detector elements extending in said scanning direction.

Preferably, the successive increments of detector array scanning motion are about one half of the dimension of a single detector element in the scanning direction.

This geometry improves the resolving capability of the detector array in the y direction (parallel to the columns) to the limit of its resolving power as dictated by the aperture cutoff frequency determined by element size. The geometry modifies the Nyquist frequency of the detector in the y direction to a value which is substantially coincident with the aperture cutoff frequency, rather than permitting a Nyquist frequency more limiting in resolution than the aperture cutoff criterion.

Resolution of the detector in the x direction (perpendicular to the columns) is improved by employment of novel time delay and integrate circuitry and by provision for its operation in a novel sequence. More specifically, the TDI circuitry samples detector element outputs at successive increments of detector motion less than, e.g. equal to half of, the width of a single detector element in a direction perpendicular to the column, i.e., parallel to the detector element rows. This actual reduction in sampling distance improves the Nyquist frequency in the x direction, rendering it substantially equal to the aperture cutoff frequency, so that the Nyquist frequency does not limit resolution to a level poorer than that permitted by the aperture cutoff limitation.

Preferably the time delay and integrate circuitry operates in a mode wherein the total accumulated charge of a given image pixel accumulated in a given detector element is sampled only at successive increments of detector motion equal to detector motion over a distance equal to twice the width of a single detector element. The time delay and integrate circuitry may also comprise circuitry for interposing delay on the total accumulated charge of each said element, with respect to the total accumulated charge of the immediately preceding element, equal to the time required for the detector array to move a distance equal to twice the width of a single detector element.

One radiography system in accordance with the invention will now be described, by way of example, with reference to the accompanying drawings in which:-

Figure 1 is a perspective view of a digital radiography system incorporating the present invention;

Figure 2 is a plan view, shown partially in graphical and partially in block form, of a digital radiography system incorporating the present invention;

Figure 3 is an elevational view, partially broken away, illustrating a component of a prior art digital radiography system;

Figure 4 is a graphical illustration of an operative sequence associated with use of the component illustrated in Figure 3;

Figure 5 is an elevational view, partially broken away, illustrating a portion of a component of the system illustrated in Figures 1 and 2;

Figure 6 is a graphical illustration of an operative sequence associated with the use of the component illustrated in Figure 5;

Figure 7 is a graphical illustration of a component of the system of Figures 1 and 2, illustrating a particular form of mathematical notation interpretive of a portion of the present disclosure, and

Figure 8 is a graphical illustration showing a sequence of operation of components of the system of Figures 1 and 2, including the component illustrated in Figure 5.

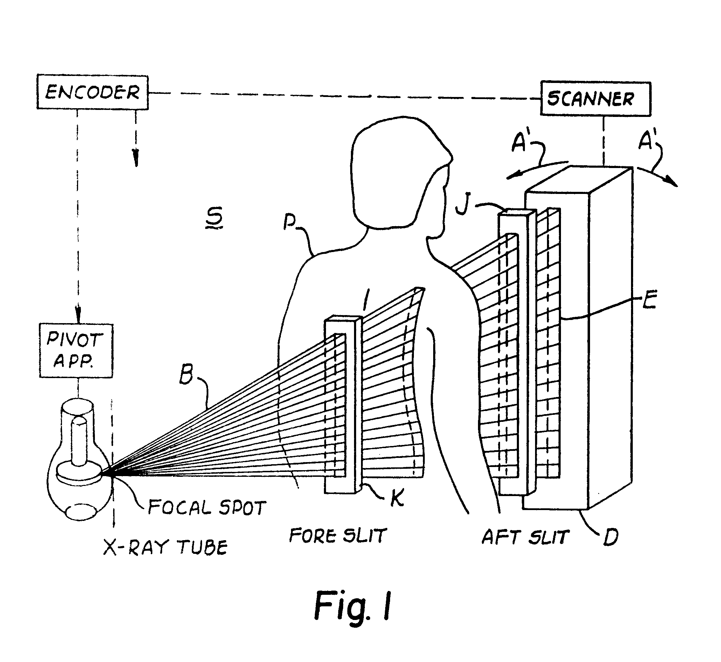

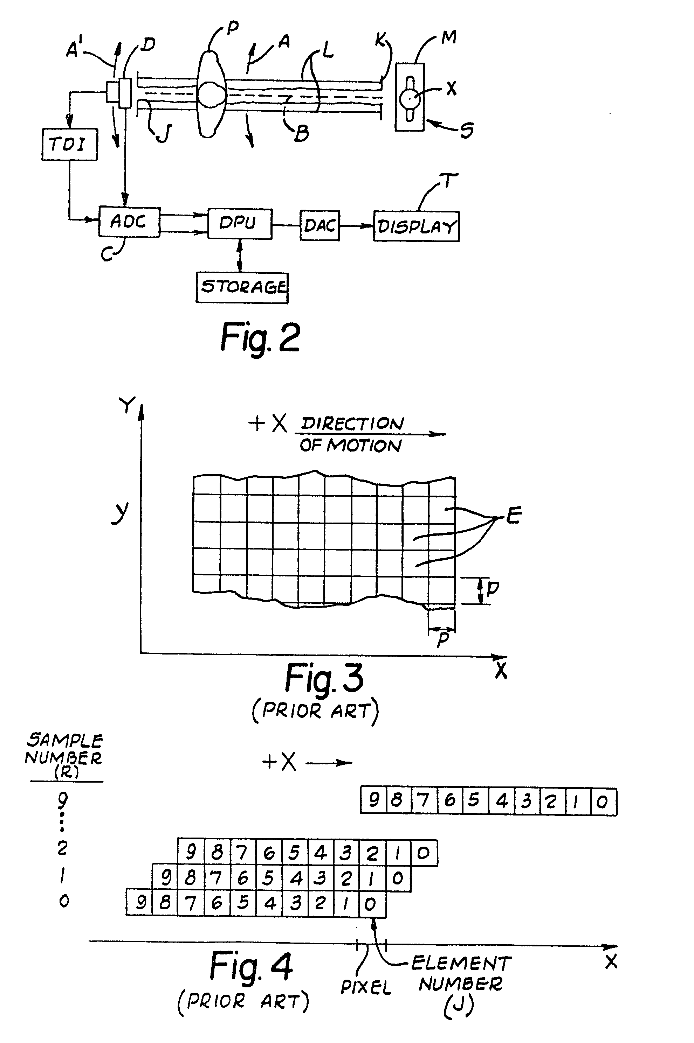

Figures 1 and 2 illustrate a slit projection type of digital radiography system S in which the present invention is incorporated. The system S scans an X-ray spread beam approximately 1 to 2 centimeters in thickness about a vertical axis across a patient's chest and detects a pattern of X-rays emergent from the patient's body. Information represented by the detected X-rays is processed and displayed to illustrate a representation of an image of the patient's internal body structure or condition.

More specifically, the system S includes an X-ray source X affixed to mounting structure M for projecting the spread beam B of X-rays through the body of a patient P, to strike an aligned detector assembly D comprising a plurality of detector elements. The spread beam B is confined by a forward slit K to a substantially vertical plane. The detector assembly D comprises a generally vertically elongated staggered array of individual detector elements E (described in more detail below) and is aligned with the vertical plane defined by the spread beam B. An aft slit J attached to the detector assembly D serves to further define the spread beam B.

The X-ray source X is mounted on the structure M to rotate about a vertical axis, defined in Figure 2 as extending into the plane of the paper. Mechanical linkage L couples the X-ray tube X to the detector array D and slits K and J and causes the detector array to scan behind the patient's body along an arcuate path defined by the arrows A, A' in order to maintain the detector assembly D aligned with the beam B throughout the scanning rotative motion of the beam.

The embodiment of the scanning mechanism is not to be limited to fixed or rigid mechanical linkage connecting the elements to be moved. Servo control and associated power drive apparatus embodiments can also be adapted by those of skill in the art to accomplish the desired scanning.

In accordance with another aspect of this embodiment, the X-ray tube X can also be pivoted about its focal spot, to maintain the beam B aligned with the scanning detector.

The X-ray source X is controlled by power means to emit the spread beam B as either a continuous X-ray beam or a rapid succession of X-ray pulses. The X-ray tube X and the detector assembly D synchronously scan, about a vertical axis, across the patient from one side of his body to the other. Analog detector outputs from each of the detector elements are periodically sampled. Each sampling produces analog signals representing a portion of image information. Over the course of the scan from one side to the other side, signals are developed describing a plurality of image lines, which together constitute an area image of the patient's internal body structure.

The analog signals produced by the detector assembly are provided to an analog to digital converter C which digitizes the outputs and feeds them to a digital processing and receiving unit DPU. The DPU processes these digitized output signals to construct a digital representation of an image of the patient's internal body structure scanned by the X-ray beam B, on a pixel-by-pixel basis. Digital signals from the DPU are converted to analog form by way of a digital to analog converter DAC, and fed to a display unit T, which, in response, produces an image in visual form corresponding to the image representing signals from the DPU.

Optionally, digital storage means can be provided in conjunction with the DPU in order to digitally store the image representations for future use. In such event, the digitally stored signals can be later played through the DPU, converted to analog form, and their corresponding images then displayed.

Coupled to each of the elements E (see Figure 3) of the detector assembly D is time delay and integrate circuitry TDI. The time delay and integrate circuitry operates to shift and add analog signals from the detector elements E to produce other analog signals representing the data from the detector elements E possessing an improved signal-to-noise ratio. As pointed out above, a form of prior art TDI circuitry employed in digital radiography is described in US Patent No. 4,383,327.

A significant aspect of the present invention involves the configuration of the array of detector elements, and improvements in the structure and mode of operation of the TDI circuitry associated therewith. Understanding of the present invention is facilitated, however, by an explanation of the prior art detector configuration and corresponding sampling circuitry operation of the prior art.

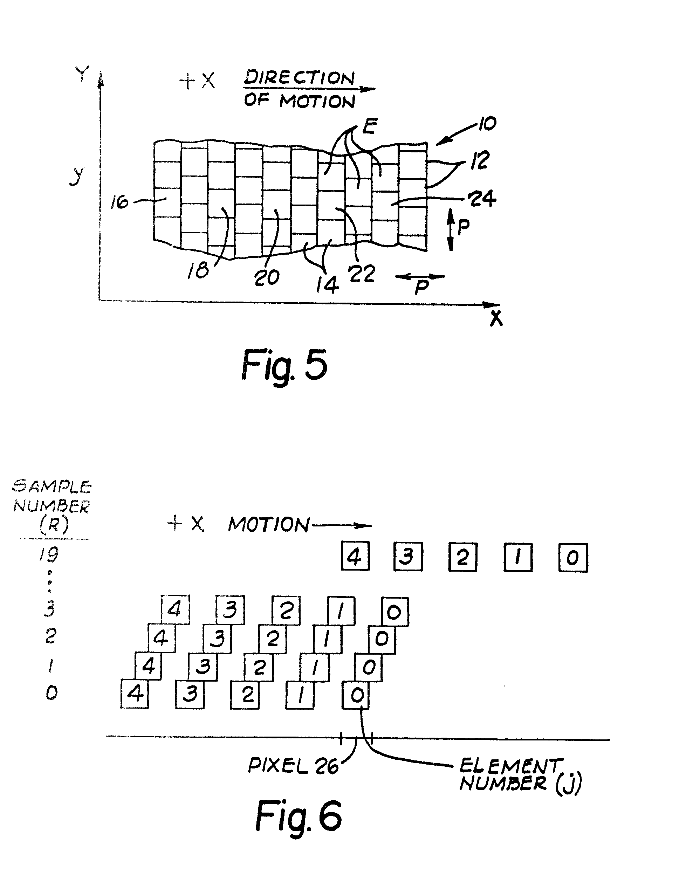

The shifting and adding required for a prior art rectangular matrix of square detector elements (Figure 3) is illustrated in Figure 4. From the frame of reference of a patient, the detector array moves, to the right as shown in Figure 3, at a constant speed v in the x direction. From the frame of reference of the detector array D, the patient moves in the -x direction with speed -v.

Each of the detector elements (as shown at E in Figure 3, for example) integrates the X-ray signal received, represented as accumulated electric charge, until it is sampled. If the detector elements are sampled every t seconds, in the patient's reference, the sampling distance d (in the x direction) is defined by the equation d = vt.

Consider a single row of ten detector elements extending in the x direction, as shown in Figure 4. Let the expression s(j,k) represent the charge accumulated at the k-th sampling of the j-th detector element, where the elements are numbered from right to left and j ranges from 0 to 9. Assume that the sampling is adjusted so that d is the same as the detector element center-to-center spacing p (the "pitch").

Consider the patient "pixel" located in alignment with the element 0 at sample 0. This pixel will be facing element 1 at sample 1, element 2 at sample 2, and so on. Hence the final value Q of the charge signal ultimately shifted from element 9, representing the X-ray value of the pixel in question, is given by the expression:

$$Q = s(0,0) + s(1,1) + ... + s(9,9). \quad \text{(Eq.1)}$$

Those of skill in the art will recognize that this equation describes a shift-and-add sequence. Time delay and integration (TDI) utilizing CCD (charge coupled device) analog shift registers is known to be appropriately suited to perform this shift-and-add sequence in the analog domain, as described in US-A-4,383,327.

The prior art detector illustrated in Figure 3 comprises a rectangular matrix of square detector elements having sides of length p. The spatial frequency response of this detector is the product of the x and y responses, i.e.:

$$MTF = (MTFx)(MTFy) \quad \text{(Eq.2)}$$

Also,

$$MTFx = MTFy = \text{sinc}(pf), \quad \text{(Eq.3)}$$

where f is the spatial frequency. The x and y modulation transfer functions each have their first zero at f = 1/p. This is referred to here as the aperture cutoff frequency.

In the y direction, the sampling distance is simply the detector element pitch p.

In the x direction, the sampling distance d depends upon the sample interval t. In such prior art TDI applications, t must be chosen so that d equals p, as in the above example. The Nyquist frequency due to sampling is then 1/(2p) in both directions. Therefore, the Nyquist frequency is only half the detector aperture cutoff frequency. This means that the spatial resolution of the detector, in this prior art example, is poorer than that dictated by the detector element aperture, and aliasing artefacts will be present in an image derived from such a detector as explained above. Note that the pixel pitch (sampling pitch) is the same as the detector element pitch p.

The facts and theory described in connection with above prior art are within the scope of knowledge of one of ordinary skill in this art. For the benefit of one of those, however, who may not be conversant with the relevant art, the principles involved in the foregoing analysis are explained in the following publications: Giger, M.L., et al "Investigation of Basic Imaging Properties in Digital Radiography: Modulation Transfer Function" Medical Physics 11(3) May/June 1984, at pages 287-295;

"A Method to Measure the MTF of Digital X-Ray Systems" Sones, R.A. et al Medical Physics 11(2) March/April 1984 at pages 166-172;

Goodman, J.W. "Introduction to Fourier Optics", McGraw-Hill 1968, at pages 21-25;

Newton, J.H. et al , "Radiology of the Skull and Brain", Technical Aspects of Computed Tomography, Volume 5, pp. 3931, 3958.

The present invention eliminates the discrepancy in resolving power of the detector between the inherent detector element aperture cutoff and the Nyquist frequency restriction. The present invention enables obtaining the maximum theoretical resolution inherently possible within the aperture cutoff limitation on resolution. This advantage is obtained consistent with full maintenance of the advantages of time delay and integration circuitry.

Referring to Figure 5, the detector matrix 10 of the present invention is represented as a staggered array of square detector elements arranged in horizontal rows such as indicated at 12 and vertical columns such as indicated at 14. Each column 14 is vertically displaced or offset from adjacent columns by a distance equal to one half p, i.e., one half the side dimension of each square detector element.

In this configuration, the modulation transfer function (MTF) is still given by the relation expressed in equations 2 and 3 above.

In the preferred embodiment, the radiation-sensitive square faces of the detector elements are approximately 0.35 millimeters on a side. The staggered detector array includes 30 columns and 2016 rows having an overall dimension of approximately 10.5 by 352.8 millimeters.

Each detector is of a known type, such as a photodiode coupled to an X-ray scintillator, which produces an analog charge signal in response to receipt of radiation incident on its sensitive surface. This charge is integrated during the time that the element's radiation sensitive face is exposed to the radiation.

Time delay and integrate circuitry is coupled to each row of detector elements. As the detector moves in the direction given by the arrow in Figure 5 relative to the patient, the TDI circuitry samples, delays and adds charge in a unique sequence, to enable great improvement in resolution in conjunction with the staggered aray of detector elements. Each accumulation step is preceded by a sampling step.

The Nyquist frequency is doubled in the y direction by stagering the detector elements. The Nyquist frequency is doubled in the x direction by sampling every detector half-width increment, in conjunction with use of the staggered array.

In the present embodiment, the sampling takes place at a frequency such that a sampling occurs with each successive incremental relative movement of the detector by a distance of p/2. That is, the time delay and integrate circuitry samples the charge packets present at the detector element outputs along a row each time the detector array has moved a distance equal to half the length of a side of one of the square detector elements.

The specific operation of the TDI circuitry will be discussed in connection with Figure 6.

It is important to define the meaning of a "row" of detectors as illustrated in Figure 5. A "row" of detector elements extending in the x direction as in Figure 5 is exemplified by the set of elements 16, 18, 20, 22, 24 as illustrated in that figure. Thus, the centre-to-centre spacing of elements in a given row extending in the x direction is 2p, or twice the lateral dimension of a side of a single one of the uniform detector elements.

In the y direction as shown in Figure 4, the sampling distance dictated by the staggered array geometry is p/2, because the rows are partially interspersed. This sampling distance yields a Nyquist sampling cutoff at f = 1/p, which is equal to the aperture cutoff frequency. Thus, in this preferred embodiment, the actual resolution obtainable in the y direction is now limited only by the aperture cutoff frequency, which, as described above, is twice as favourable as that dictated by the Nyquist spatial frequency which was obtained in the prior art rectangular array system.

The same coincidence between the limiting resolution of the Nyquist frequency and that of the aperture cutoff is achieved in the x direction by choosing the sampling interval t in the x direction so that the sampling incremental distance of detector relative motion is p/2.

This improvement results because the Nyquist frequency doubles as the sampling distance is halved. For a sampling distance of p the Nyquist frequency $f_n = 1/(2p)$. When p is replaced with p/2, the expression becomes $f_n = 1/[2 (p/2)]$, or $f_n = 1/p$.

Therefore, for a predetermined detector element size the configuration of the present embodiment provides twice the resolution, in both directions, of that of the prior art. Pixel size in the present embodiment is only one quarter of the area of one detector element.

Moreover, the embodiment of this invention substantially reduces aliasing, compared to that of the standard prior art rectangular array.

In order to achieve these improvements, the staggered detector array requires associated time delay and integrate circuitry that differs from the circuitry whose operation is exemplified in equation 1. Consider a single row of detector elements in Figure 5 extending in the x direction, and comprising five elements. Let s(j,k) represent the k-th sample of the j-th element, where the elements are numbered from right to left and j ranges from 0 to 4 (see Figure 6). Consider also the image portion or pixel which is designated by the reference character 26 and which is aligned with element 0 at sample 0. Since the sampling distance in this embodiment is p/2, this particular pixel will be aligned with element 1 only at the fourth sampling, with element 2 at sample 8, element 3 at sample 12 and so on. Therefore, the final value generated by the time delay and integration circuitry and corresponding to this pixel will be:

$$S_0 = s(0,0) + s(1,4) + ... + s(4,16). \quad (4a)$$

Analogous equations apply for other pixels which are aligned with element 0 at sample times 1, 2 and 3:

$$S_1 = s(0,1) + s(1,5) + ... + s(4,17). \quad (4b)$$

$$S_2 = s(0,2) + s(1,6) + ... + s(4,18) \quad (4c)$$

$$S_3 = s(0,3) + s(1,7) + ... + s(4,19). \quad (4d)$$

Generalizing the relationships expressed by equations 4a-4d, the total accumulated charge corresponding to the j-th pixel of the i-th row i.e., P(i,j), of the total derived image is given by the following expression:

$$P(i,j) = \sum_{k=0}^{N-1} d(i, k, 4k+j) \quad (Eq.5)$$

where:

d(m,n,k) = the charge accumulated in the n-th detector element of the m-th row during the k-th sampling period.

and N = the number of detector elements per row.

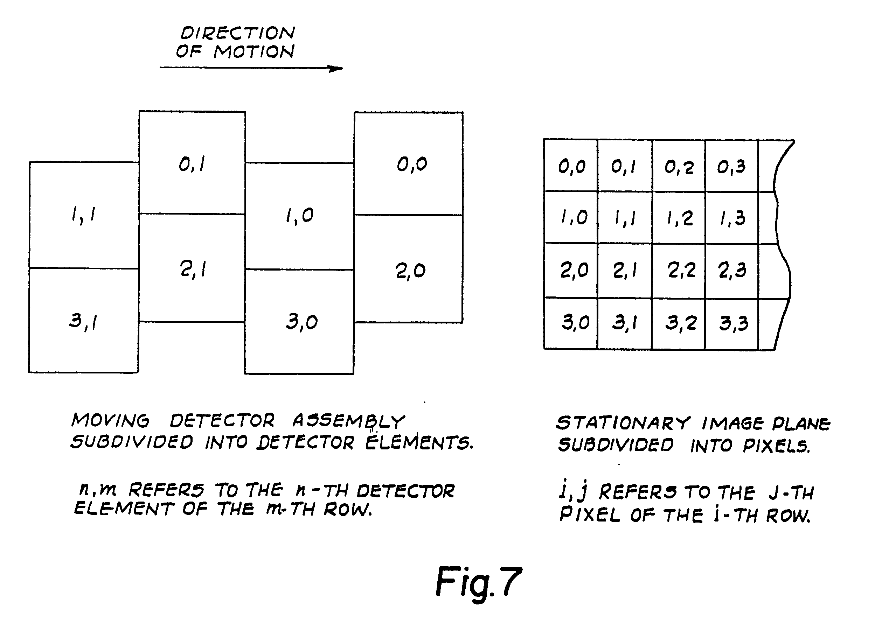

See Figure 7 for a graphical representation of the notation of equation (5).

The shift and add sequence represented by equations 4 and 5 can be implemented in a variety of ways. A preferred implementation involves the use of CCD analog shift registers in a time delay integration configuration. It is important to note, however, that operating in this sequence, the TDI CCD cells cannot themselves constitute a portion of the detector elements. Rather, the detector elements must be separate from, but coupled to, the TDI circuitry.

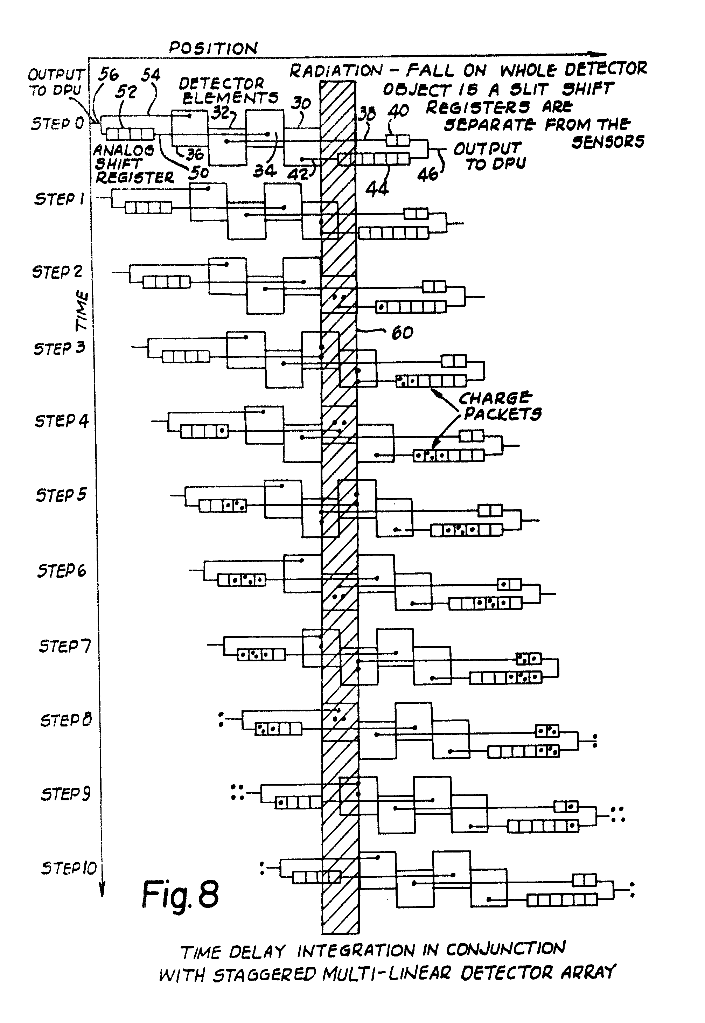

Figure 8 illustrates the structure and operation of circuitry for implementing the time delay and integrate sequencing represented by equations 4 and 5. Figure 8 illustrates a portion of a detector array at eleven different steps of relative detector motion with respect to a patient, these steps being labeled from 0-10.

As shown in the illustration corresponding to step 0, two rows of detector elements are illustrated, there being two detector elements in each row, having their center-to-center spacing equal to 2p, where p is the dimension of one side of each of the square elements. Referring to the illustration of step 0, one row of such elements is designated 30, 32, while the other row is designated 34, 36.

Detector element 32 is coupled by a lead 38 to a two-cell shift register 40. Detector element 30 is coupled by a lead 42 to a six element shift register 44. The outputs of the registers 40, 44 are connected in parallel to an output line 46. The output line 46 receives analog signals from the registers 40, 42 representing total charge value corresponding to a particular pixel or image portion which has been "read" by both of the detector elements 30, 32 in the detector element row of interest. Output lead 46 is coupled as an input to the ADC.

The analog output of detector element 34 is coupled by a lead 50 to a four-cell shift register 52. The output of detector element 36 is coupled by a lead 54 directly in parallel with the output of the shift register 52, the resultant output being summed and appearing at an output lead 56. Analog signals appearing at the lead 56 are transmitted to the DPU for further processing in accordance with known techniques. Each output appearing at the lead 56 is an analog signal representing a total pixel value of an image portion which has been read by both the detector elements in the associated row, i.e., elements 34, 36.

Each of the leads connected directly to elements 30, 32, 34, 36 comprises a translucent polysilicon conductor.

It is important to understand that the signals at the leads 46, 56 are not mixed by the system, because they are signals representing different rows of data in the total image, elements 34, 36 representing an upper row and elements 30, 32 representing a lower row.

Also illustrated in Figure 8 is a shaded band of radiation 60. The radiation band 60 is to be considered as falling upon whatever detector element(s) are illustrated as within the band at any given step. In practice, radiation from the spread beam is continuously directed through a patient toward all of the detector elements simultaneously, the radiation falling upon individual detector elements in greater or lesser amounts depending on the patient's internal body structure. For purposes of clarity of illustration, however, the radiation pattern emergent from the patient will be considered to be a slit having a width equal to that of the radiation band 60 illustrated in Figure 8.

Also for purposes of clarity, the reference characters illustrated in conjunction with the illustration corresponding to step 0 are omitted for the other steps, in order to avoid undue cluttering of the drawing. The reference characters associated with the illustration of step 0 are to be considered as applicable also to the corresponding portions of the illustrations of the subsequent steps 1-10.

The operation of the circuitry and apparatus in Figure 8 is as follows.

At step 0, the radiation band 60 is not incident on any portion of any of the detector elements. Therefore, none of the detector elements generate any charge packets constituting analog signals representing incident radiation.

It is be understood that clocking circuitry, of known type but not illustrated specifically in Figure 8, is provided. At each step increment, the clocking circuitry causes the signals at the leads 38, 42, 54 and 50 to be sampled. With respect to the leads 38, 42 and 50, which are coupled respectively to shift registers 40, 44 52, the clocking circuitry causes a signal representing the respective charge packet from the associated element to be stored in the first cell of the corresponding shift register. The clocking signal also causes any previously stored charge related signals in each of the shift registers to shift to the next subsequent sell of that register. The clocking signal causes any signal stored in the ultimate cell of a shift register to proceed to the associated one of outputs 46, 56.

The clocking circuitry executes its ensemble of operation as described above with a frequency such that the elements are sampled at successive increments of p/2 detector travel.

In the portion of Figure 8 designated step 1, the detector has moved to the right, relative to the patient, a distance of p/2, or half the width of a detector element. At this point the detector element 30 is positioned such that half the detector element receives incident radiation from the slit radiation pattern 60. The element 30 generates a charge "packet" which is designated by a dot located in the center of that element. The other elements, not being within the radiation slit 60, generate no charge packets.

By the time the detector portion has reached step 2 of its incremental motion, the charge packet represented by the dot in the illustration of step 1 has been clocked into the first cell of the register 44. The element 30 is now completely illuminated with incident radiation from the slit pattern 60. Since the detector element generates charge packets in accordance with the amount of radiation received, this element produces an amount of charge at its output equal to two of the packets designated by the dot in step 1. This generated charge is represented by two dots within element 30 as illustrated in connection with step 2.

Upon arrival at the location illustrated in connection with step 3, the double charge packet produced by element 30 is clocked into the first cell of the register 44. The single charge packet formerly located in the first cell of register 44 is clocked into the second cell of that register. Meanwhile, in a fashion analogous to that described above, both elements 30 and 34 generate single charge packets.

This progression of generation and storage of charge packets continues. Note that it is not until step 6 that any charge from the element 32, in the same row as element 30, is clocked into the two-cell register 40. It will be recalled that charge, in this illustration, was first clocked into the register 44 at step 2, four steps previous to the first appearance of charge signal in the register 40. Thus, charge clocked into the register 44 is delayed by four p/2 increments of relative detector motion, with respect to charge in the register 40.

It will also be seen from a review of equations 4 and 5 that the circuitry configuration and operating sequence of Figure 8 implements the operations described in those equations. More specifically, the Figure 8 configuration implements the operations described in those equations wherein signals from each element of a detector element row are combined with signals from the immediately following element in the row only after a delay of four sampling steps.

Accordingly, the operations described in equations 4 and 5 are implemented by providing each detector element in a row with a delay which is different and independent from the delay imposed on the outputs of other cells in the row. In this way, the prescribed delay sequences for implementing equations 4 and 5 can be built into the TDI circuitry coupled to the respective elements.

Referring again to Figure 8, step 8, it will be seen that at this point sufficient steps have taken place such that the output of data to the DPU has begun. Step 8 corresponds to an output representing two charge packets at the lead 46. The signal corresponding to two charge packets is derived from the summation of the two single charge representing signals present in the ultimate cells of each of the shift registers 44, 40.

At step 9, the clocking circuitry produces an output representing four charge packets, the result of the

9

summation of the two signals each representing two charge packets present in the ultimate cells of the registers 44, 40 at step 8.

As the signals representing an image pixel read by the elements 30, 32 are produced at the output 46, analogously produced signals are generated at the output 56 which corresponds to the upper row of elements, i.e., elements 34, 36.

A relative delay in output signals from the element 34, with respect to those from the element 36, is effected by the presence of the four-cell shift register 52 in the line 50, and by the tact that the output from the element 36 is presented directly over the lead 54 to the output 56.

It will be observed that, by the use of the TDI circuitry described in connection with Figure 8, the signal-to-noise ratio of the output signals describing the respective image pixel is enhanced. If the TDI circuitry illustrated in Figure 8 were not used, the output of, for example, element 30, in passing through the radiation slit 60 would be a succession of three signals representing 1, 2 and 1 charge packet, respectively, which would be emitted at steps 1, 2 and 3. By the use of TDI circuitry, in connection with which the image pixel is defined by the sum of responses of the respective elements reading or viewing the pixel, the corresponding outputs are signals representing 2, 4 and 2 charge packets, occurring at steps 8, 9 and 10.

Design of techniques for producing imaging data from sequentially occurring line-representing sets of data from a photodiode array, such as produced here, are within the scope of ordinary skill in the art, as evidenced by publications including United States Patent No. 4,203,037, to Gur et al, and by the several patents and publications identified therein.

Figure 8 constitutes an illustration of the operation and components of the TDI circuitry in a simplified environment, i.e., considering only two rows of two detector elements each. Those of skill in the art will easily be able to expand this illustration in an analogous fashion to the use of larger numbers of elements in each row, and to larger numbers of rows. For example, if three elements were employed in each row, the right hand element in Figure 8 would have its output coupled to a ten-cell shift register. The middle element will be coupled to a six-cell register and the left hand, or third element would be coupled to a two-cell register. This configuration would maintain the respective delay relationships in summing signals from the respective elements as expressed in equations 4 and 5 above.

It is to be understood that the description of this embodiment of the invention is intended to be illustrative, rather than exhaustive, of the invention. Those of ordinary skill in the relevant art will be able to make certain additions, deletions and modifications to the described embodiment of the invention, without departing from the scope of the invention, as described in the following claims.

**Claims**

1. A radiography system comprising: a radiation source (X); a detector array (D) comprising a plurality of rows (12) of uniform detector elements (E) interspersed so as to define a plurality of columns (14) perpendicular to said rows (12) and arranged in a staggered pattern, each detector element (E) being responsive to radiation incident thereon for producing an electrical output signal indicative of said incident radiation; means (L, M) for mounting said source (X) and said detector array (D) spaced sufficiently from one another to accommodate a body in a space therebetween; mechanism (L, M) for scanning said detector array (D) relative to said body when located in said space, in a direction substantially parallel to said rows (12); means for actuating said radiation source (X) to propagate radiation from the source (X) and toward said detector array (D), said radiation passing through a portion of said body located in said space, and time delay and integrate circuitry (TDI) coupled to said detector elements (E) and separate therefrom for producing an image of internal body structure in response to said electrical signals produced by said detector elements (E), said circuitry (TDI) comprising sampling circuitry for sampling said detector element electrical signals at successive increments of detector array scanning motion characterised in that said successive increments are less than a distance equal to a dimension of one of said uniform detector elements (E) extending in said scanning direction.

2. A system according to Claim 1 wherein said detector array (D) comprises at least three said columns (14) of said detector elements (E).

3. A system according to Claim 1 or Claim 2 wherein the centre-to-centre spacing between successive elements (E) along the or each said row (12) is approximately twice said dimension.

4. A system according to Claim 3 wherein said successive increments are increments of scan motion of about one half of said dimension.

5. A system according to Claim 3 or Claim 4 wherein said time delay and integrate circuitry (TDI) implements a time delay and integrate function defined by the following relationship:

$$P_{(i,j)} = \sum_{k=0}^{N-1} d(i,k,4k + j)$$

where:

$P_{(i,j)}$ = the total accumulated charge of the j-th pixel of the i-th row of said image;

d(m,n,k) = the charge accumulated in the n-th detector element of the m-th row in the k-th sampling period, and

N = the number of elements per row.

6. A system according to any one of Claims 1 to 5 wherein said time delay and integrate circuitry (TDI) operates in a mode wherein the total accumulated charge of a given image pixel accumulated in a given detector element (E) is sampled only at successive increments of detector motion equal to detector motion over a distance equal to twice the width of a single detector element (E).

7. A system according to Claim 6 when dependent on Claim 4 wherein said time delay and integrate circuitry (TDI) comprises circuitry for interposing delay on the total accumulated charge of each said element (E), with respect to the total accumulated charge of the immediately preceding element (E), equal to the time required for the detector array (D) to move a distance equal to twice the width of a single detector element (E).

8. A system according to any one of Claims 1 to 7 wherein said time delay and integrate circuitry (TDI) comprises: means for sampling said detector element output signals at successive increments of detector motion equal to approximately one half the width dimension of an individual detector element taken parallel to one of said rows; means for delaying respective output signals from detector elements of one of said rows in different amounts in order to present said output signals of said elements of said row pertaining to a single image pixel simultaneously at an output; means for summing said simultaneously presented output signals, and means for processing said summed signals to obtain representation of an image of internal subject structure.

9. A system according to Claim 8 wherein said detector array comprises a row of uniform square detector elements having centres spaced by 2p, where p is the dimension of an element side; and said time delay and integrate circuitry (TDI) means for sampling detector element outputs with each p/2 of successive scanning motion increment; means for accumulating, as a representation for a given image portion, a set of only each fourth sampling of each detector output, and means for accumulating as a representation for another given image portion, a different set of fourth samplings offset in time from said set.

10. A system according to any preceding claim wherein said scanning means (L, M) comprises means for effecting pivotal motion of said source in synchronism with detector motion to maintain constant alignment between the detector and the radiation emanating from said source.

11. A system according to any precding claim wherein said scanning means (L, M) further comprises means for moving said detector array along an arcuate path.

12. A system according to any one of the preceding claims wherein said radiation is x-radiation.

## Revendications

1. Installation radiographique, comprenant une source (X) d'un rayonnement, une barrette détectrice (D) comprenant plusieurs lignes (12) d'éléments détecteurs uniformes (E) disposés afin qu'ils forment plusieurs colonnes (14) perpendiculaires aux lignes (12) et disposées avec un dessin décalé, chaque élément détecteur (E) étant sensible au rayonnement qui lui parvient et étant destiné à créer un signal électrique de sortie représentatif du rayonnement incident, un dispositif (L, M) de montage de la source (X) et de la barrette détectrice (D) à distance mutuelle suffisante pour la disposition d'un corps dans l'espace intermédiaire, un mécanisme (L, M) destiné à balayer la barrette détectrice (D) par rapport au corps, placé dans l'espace, en direction sensiblement parallèle aux lignes (12), un dispositif de commande de la source (X) du rayonnement afin que le rayonnement de la source (X) soit dirigé vers la barrette détectrice (D), le rayonnement passant à travers une partie du corps placé dans l'espace, et un circuit à retard et d'intégration (TDI) couplé aux éléments détecteurs (E) et séparé de ceux-ci afin qu'il forme une image de la structure interne du corps en fonction des signaux électriques produits par les éléments détecteurs (E), le circuit (TDI) comprenant un circuit d'échantillonnage des signaux électriques des éléments détecteurs pour des quantités élémentaires successives du mouvement de balayage de la barrette détectrice, caractérisée en ce que les quantités élémentaires successives sont inférieures à une distance égale à la dimension de l'un des éléments détecteurs uniformes (E) dans la direction de balayage.

2. Installation selon la revendication 1, dans laquelle la barrette détectrice (D) comporte au moins trois colonnes (14) d'éléments détecteurs (E).

3. Installation selon la revendication 1 ou 2, dans laquelle l'espacement des centres des éléments successifs (E) le long de la ligne ou de chaque ligne (12) est à peu près égal au double de ladite dimension.

4. Installation selon la revendication 3, dans laquelle les quantités élémentaires successives sont des quantités élémentaires de déplacement de balayage égales à la moitié environ de ladite dimension.

5. Installation selon la revendication 3 ou 4, dans laquelle le circuit à retard et d'intégration (TDI) exécute une fonction à retard et d'intégration représentée par la relation suivante :

$$P_{(ij)} = \Sigma_{k=0}^{N-1} d(i,k,4k+j)$$

$P_{(i,j)}$ étant la charge totale cumulée dans le $j^{ième}$ élément d'image de la $i^{ième}$ ligne de l'image, $d(m,n,k)$ étant la charge cumulée dans le $n^{ième}$ élément détecteur de la $m^{ième}$ ligne dans la $k^{ième}$ période d'échantillonnage, et N étant le nombre d'éléments par ligne.

6. Installation selon l'une quelconque des revendications 1 à 5, dans laquelle le circuit à retard et d'intégration (TDI) travaille dans un mode dans lequel la charge totale cumulée d'un élément d'image donné, accumulée dans un élément détecteur donné (E), est échantillonnée uniquement pour des quantités élémentaires successives de déplacement du détecteur qui sont égales au déplacement du détecteur sur une distance égale au double de la largeur d'un seul élément détecteur (E).

7. Installation selon la revendication 6, dépendant de la revendication 4, dans laquelle le circuit à retard et d'intégration (TDI) comprend un circuit destiné à introduire un retard dans la charge totale cumulée de chacun des éléments (E) par rapport à la charge cumulée totale de l'élément immédiatement précédent (E) qui est égal au temps nécessaire pour que la barrette détectrice (D) se déplace d'une distance égale au double de la largeur d'un élément détecteur unique (E).

8. Installation selon l'une quelconque des revendications 1 à 7, dans laquelle le circuit à retard et d'intégration (TDI) comporte un dispositif d'échantillonnage des signaux de sortie des éléments détecteurs pour les quantités élémentaires successives de déplacement des détecteurs égales à la moitié environ de la dimension en largeur d'un élément détecteur individuel, parallèlement à l'une des lignes, un dispositif destiné à retarder les signaux respectifs de sortie des éléments détecteurs de l'une des lignes de différentes quantités afin que les signaux de sortie des éléments de la ligne, appartenant à un seul élément d'image, soient présentés simultanément à une sortie, un dispositif destiné à ajouter les signaux de sortie présentés simultanément, et un dispositif de traitement des signaux ajoutés, destiné à l'obtention de la représentation d'une image d'une structure interne du sujet.

9. Installation selon la revendication 8, dans laquelle la barrette détectrice comprend une ligne d'éléments détecteurs carrés uniformes ayant des centres séparés par une distance 2p, p étant la dimension du côté d'un élément, et le circuit à retard et d'intégration (TDI) est destiné à échantillonner les signaux de sortie des éléments détecteurs pour chaque quantité élémentaire de déplacement de balayage successif égale à p/2, un dispositif étant destiné à accumuler, comme représentation d'une partie donnée de l'image, un jeu de chaque quatrième échantillonnage de chaque signal de sortie du détecteur, et un dispositif étant destiné à cumuler, comme représentation d'une autre partie donnée d'image, un jeu différent de quatrièmes échantillonnages décalés dans le temps par rapport au premier jeu.

10. Installation selon l'une quelconque des revendications précédentes, dans laquelle le dispositif de balayage (L, M) comporte un dispositif destiné à assurer le pivotement de la source en synchronisme avec le mouvement du détecteur afin qu'un alignement constant soit conservé entre le détecteur et le rayonnement provenant de la source.

11. Installation selon l'une quelconque des revendications précédentes, dans laquelle un dispositif de balayage (L, M) comporte en outre un dispositif destiné à déplacer la barrette détectrice le long d'un trajet courbe.

12. Installation selon l'une quelconque des revendications précédentes, dans laquelle le rayonnement est un rayonnement X.

## Patentansprüche

1. Durchleuchtungssystem, aufweisend: eine Strahlungsquelle (X); ein Detektorfeld (D), das mehrere Reihen (12) gleichförmiger Detektorelemente (11) aufweist, die so verschachtelt sind, daß sie mehrere Spalten (14) definieren, die senkrecht zu den Reihen (12) sind und in einem versetzten Muster angeordnet sind, wobei jedes Detektorelement (E) auf es auftreffende Strahlung anspricht, um ein elektrisches Ausgangssignal zu erzeugen, das die einfallende Strahlung anzeigt; Einrichtungen (L, M) zum Anbringen der Quelle (X) und des Detektorfeldes (D), die ausreichend voneinander beabstandet sind, um einen Körper in einem Raum dazwischen aufzunehmen; einen Mechanismus (L, M) zum Abtasten des Detektorfeldes (D) relativ zu diesem Körper, wenn dieser in diesem Raum angeordnet ist, in einer Richtung im wesentlichen parallel zu den Reihen (12); Einrichtungen zum Betätigen der Strahlungsquelle (X), um Strahlung von der Quelle (X) und auf das Detektorfeld (D) hin auszusenden, wobei die Strahlung durch einen Teil des in diesem Raum angeordneten Körpers hindurchtritt, und eine Zeitverzögerungs- und Integrationsschaltung (TDI), die an die Detektorelemente (E) angeschlossen und separat hiervon ist, um ein Bild einer internen Körperstruktur ansprechend auf die elektrischen Signale zu erzeugen, die von den Detektorelementen (E) erzeugt worden sind, wobei die Schaltung (TDI) eine Abtastschaltung zum Abtasten der elektrischen Detektorelementsignale bei aufeinanderfolgenden Inkrementen der Detektorfeldabtastbewegung umfaßt,
**dadurch gekennzeichnet,**
daß die aufeinanderfolgenden Inkremente geringer als eine Distanz gleich einer Ausdehnung eines der gleichförmigen Detektorelemente (E) ist, die sich in der Abtast richtung erstreckt.

2. System nach Anspruch 1, in welchem das Detektorfeld (D) zumindest drei dieser Spalten (14) der Detektorelemente (E) umfaßt.

3. System nach Anspruch 1 oder Anspruch 2, in welchem der Mitte-zu-Mitte-Abstand zwischen aufeinanderfolgenden Elementen (E) entlang der oder jeder solchen Reihe (12) angenähert dem Zweifachen dieser Ausdehnung entspricht.

4. System nach Anspruch 3, in welchem die aufeinanderfolgenden Inkremente Inkremente einer Abtastbewegung von etwa einer Hälfte dieser Ausdehnung sind.

5. System nach Anspruch 3 oder Anspruch 4, in welchem die Zeitverzögerungs- und Integrationsschaltung (TDI) eine Zeitverzögerungs- und eine Integrationsfunktion ausführt, die durch die folgende Beziehung definiert ist:

$$P_{(i,j)} = \sum_{k=0}^{N-1} d(i,k,4k+j),$$

worin:

$P_{(i,j)}$ = die insgesamt angesammelte Ladung des j-ten Pixel der i-ten Reihe des Bildes;

$d(m,n,k)$ = die im n-ten Detektorelement der m-ten Reihe in der k-ten Abtastperiode angesammelte Ladung, und

$N$ = Anzahl von Elementen pro Reihe.

6. System nach einem der Ansprüche 1 bis 5, in welchem die Zeitverzögerungs- und Integrationsschaltung (TDI) in einem Modus arbeitet, worin die insgesamt angesammelte Ladung eines gegebenen Bildelements, die in einem gegebenen Detektorelement (E) angesammelt ist, nur bei aufeinanderfolgenden Inkrementen der Detektorbewegung abgetastet wird, die gleich der Detektorbewegung über eine Distanz, die dem Zweifachen der Breite eines einzelnen Detektorelements (E) entspricht, sind.

7. System nach Anspruch 6 bei Rückbeziehung auf Anspruch 4, in welchem die Zeitverzögerungs- und Integrationsschaltung (TDI) eine Schaltung, die der insgesamt angesammelten Ladung jedes solchen Elements (E) bezüglich der insgesamt angesammelten Ladung des unmittelbar vorausgehenden Elements (E) eine Verzögerung auferlegt, die gleich der Zeit ist, die für das Detektorfeld (D) notwendig ist, um sich über eine dem Zweifachen der Breite eines einzelnen Detektorelements (E) entsprechende Distanz zu bewegen, aufweist.

8. System nach einem der Ansprüche 1 bis 7, in welchem die Zeitverzögerungs- und Integrationsschaltung (TDI) aufweist: Einrichtungen zum Abtasten der Detektorelementausgangssignale bei aufeinanderfolgenden Inkrementen der Detektorbewegung, die angenähert einer Hälfte der Breitenausdehnung eines individuellen Detektorelements, parallel zu einer dieser Reihen ermittelt, entsprechen; Einrichtungen zum Verzögern der jeweiligen Ausgangssignale von den Detektorelementen einer dieser Reihen in unterschiedlichen Beträgen, um die Ausgangssignale dieser Elemente der Reihe, die zu einem einzelnen Bildelement gehören, simultan an einem Ausgang darzustellen; Einrichtungen, die die simultan dargestellten Ausgangssignale summieren und Einrichtungen, die die summierten Signale verarbeiten, um eine Darstellung eines Bildes einer internen Gegenstandsstruktur zu gewinnen.

9. System nach Anspruch 8, in welchem das Detektorfeld eine Reihe von gleichförmigen quadratischen Detektorelementen umfaßt, deren Mittelpunkte um 2p beabstandet sind, wobei p die Ausdehnung einer Elementseite ist; und die Zeitverzögerungs- und Integrationsschaltung-Einrichtungen (TDI) die Detektorelementausgänge mit jedem p/2 eines aufeinanderfolgenden Abtastbewegungsinkrements abtasten; Einrichtungen, die als eine Darstellung für einen gegebenen Bildabschnitt einen Satz von nur jedem vierten Abtastwert jedes Detektorausgangssignals ansammeln, und Einrichtungen, die als eine Darstellung eines anderen gegebenen Bildabschnitts einen anderen Satz von vierten Abtastwerten ansammeln, die von diesem Satz bezüglich der Zeit versetzt sind, aufweisen.

10. System nach einem vorhergehenden Anspruch, in welchem die Abtasteinrichtungen (L, M) Einrichtungen aufweisen, die synchron mit der Detektorbewegung eine Schwenkbewegung der Quelle bewirken, um eine konstante Ausrichtung zwischen dem Detektor und der von der Quelle ausgehenden Strahlung aufrechtzuerhalten.

11. System nach einem vorhergehenden Anspruch, in welchem die Abtasteinrichtungen (L, M) ferner Einrichtungen umfassen, die das Detektorfeld entlang eines bogenförmigen Pfades bewegen.

12. System nach einem der vorhergehenden Ansprüche, in welchem die Strahlung X-Strahlung ist.

Fig. I

Fig. 2

Fig. 3
(PRIOR ART)

Fig. 4
(PRIOR ART)

Fig. 5

Fig. 6

DIRECTION OF MOTION

STATIONARY IMAGE PLANE SUBDIVIDED INTO PIXELS.

i,j REFERS TO THE J-TH PIXEL OF THE i-TH ROW.

MOVING DETECTOR ASSEMBLY SUBDIVIDED INTO DETECTOR ELEMENTS.

n,m REFERS TO THE n-TH DETECTOR ELEMENT OF THE m-TH ROW.

Fig.7

Fig. 8

TIME DELAY INTEGRATION IN CONJUNCTION
WITH STAGGERED MULTI-LINEAR DETECTOR ARRAY